# EUROPEAN PATENT APPLICATION

(11) **EP 2 687 146 A1**
(43) Date of publication of application: **22.01.2014**
(21) Application number: 12792934.7
(22) Date of filing: 28.05.2012
(51) Int. Cl.: A61B 1/04, A61B 5/00

(54) **MEDICAL INFORMATION RECORDING DEVICE**

(30) Priority: 30.05.2011 JP 2011120856
(71) Applicant: Olympus Medical Systems Corp., Tokyo 151-0072 (JP)
(72) Inventor: KASUMI Makoto, Tokyo 151-0072 (JP)
(74) Representative: Gunzelmann, Rainer
(86) International application number: PCT/JP2012/063647
(87) International publication number: WO 2012/165381

(57) **Abstract**

A medical information recording apparatus includes: a control section that controls moving image recording of a medical image from a medical apparatus; a first recording processing section that records the medical image in a first recording region over an entire first period under the control of the control section; and a second recording processing section that, when a signal based on an operation by a user is provided in the first period, records a copy of the medical image in a second recording region in a predetermined second period before and after a timing of the operation by the user, under the control of the control section, and the medical information recording apparatus enables easy recording/reproduction of only a desired part from a medical image that has been recorded for a relatively long period of time.

## Description

### Technical Field

The present invention relates to a medical information recording apparatus that records an image obtained by a medical apparatus such as an endoscope.

### Background Art

Conventionally, endoscopes have widely been employed in, e.g., a medical field. Medical images obtained by the endoscopes are recorded on various types of media for recording diagnoses and/or cases. In recent years, along with an increase in capacity of recording media, recording of moving images from the endoscopes has come to be performed. For example, Japanese Patent Application Laid-Open Publication No. 2006-271871 discloses an apparatus that performs image recording processing according to image processing. Such recording processing for medical images is digitized and the medical images are sometimes stored in the form of a file that can be read by computers.

Furthermore, in this type of medical information recording apparatus, plural types of video images can simultaneously be recorded as moving images. For example, a combination of an endoscope and an operative field camera, rather than an endoscopic image only, enables recording of hand movements of a surgeon and an endoscopic image in association with each other. Use of such medical images enables comprehensive understanding of a treatment method, an approach of a treatment instrument and how to move the treatment instrument from the combination of an endoscopic image and hand movements, rather than an endoscopic image only, and such medical images serve as useful educational materials for further deepening doctors' understanding.

Since surgeons need to dedicate themselves to medical practice such as diagnosis, once medical practice is started, an operation related to recording of a medical image is not performed until the end of the medical practice. Accordingly, this type of medical image such as an endoscopic image or an ultrasonic image is recorded for a relatively long period of time, and reproduction of one image file often takes a long period of time.

As described above, such medical images serve as evidence showing that a proper treatment is performed, as a result of recording not only an endoscopic image but also a state of a surgeon's hands from a start to an end of a procedure. Furthermore, in addition to the above, scenes related to a procedure are specifically clipped and edited from all recorded data in an endoscopic image to utilize the scenes as a briefing material for a conference presentation, which enables a contribution to development of medical treatment techniques or medicine, and in addition, the scenes are edited with an image of hands added thereto, enabling utilization of such scenes as a learning material for young doctors.

However, in editing work, in order to clip an important scene of several minutes from a moving image of a long period of time, it is necessary to reproduce and check all recorded data from the beginning, which is an extremely large burden on users.

An object of the present invention is to provide a medical information recording apparatus capable of recording a plurality of medical images having a relatively long recoding time and easily recording only a desired part of an arbitrary medical image in the medical images.

### Disclosure of Invention

### Means for Solving the Problem

A medical information recording apparatus according to an aspect of the present invention includes: a control section that controls moving image recording of a medical image from a medical apparatus; a first recording processing section that records the medical image in a first recording region over an entire first period under the control of the control section; and a second recording processing section that, when a signal based on an operation by a user is provided in the first period, records a copy of the medical image in a second recording region in a predetermined second period before and after a timing of the operation by the user, under the control of the control section.

Also, a medical information recording apparatus according to an aspect of the present invention includes: a control section that controls moving image recording of a plurality of medical images from a plurality of medical apparatuses; a first recording processing section that records the plurality of medical images in regions for the respective medical images in a first recording region over an entire first period under the control of the control section; and a second recording processing section that, when a signal based on an operation by a user is provided within the first period, records copies of the medical images in regions for respective medical images in a second recording region in a predetermined second period before and after a timing of the operation by the user, under the control of the control section.

### Brief Description of the Drawings

Fig. 1 is a block diagram illustrating a medical information recording system in which a medical information recording apparatus according to a first embodiment of the present invention is incorporated;
Fig. 2 is a timing chart for describing recording processing for a medical image in the first embodiment;
Fig. 3 is a diagram for describing a folder created for each piece of patient information;
Fig. 4 is a flowchart for describing the first embodiment;
Fig. 5 is a diagram for describing the first embodiment;
Fig. 6 is a flowchart illustrating an operation flow employed in a second embodiment of the present invention;
Fig. 7 is a diagram for describing an image recording method in the second embodiment;
Fig. 8 is a block diagram illustrating a third embodiment of the present invention;
Fig. 9 is a diagram for describing an example of a method for creating transient images;
Fig. 10 is a flowchart illustrating an operation of the third embodiment;
Fig. 11 is a block diagram illustrating a fourth embodiment of the present invention;
Fig. 12 is a block diagram illustrating an example of a reproduction apparatus;
Fig. 13 is a diagram for describing an operation of the reproduction apparatus in Fig. 12;
Fig. 14 is a block diagram illustrating a fifth embodiment of the present invention; and
Fig. 15 is a diagram for describing the fifth embodiment of the present invention.

### Best Mode for Carrying Out the Invention

Embodiments of the present invention will be described in detail below with reference to the drawings.

### (First Embodiment)

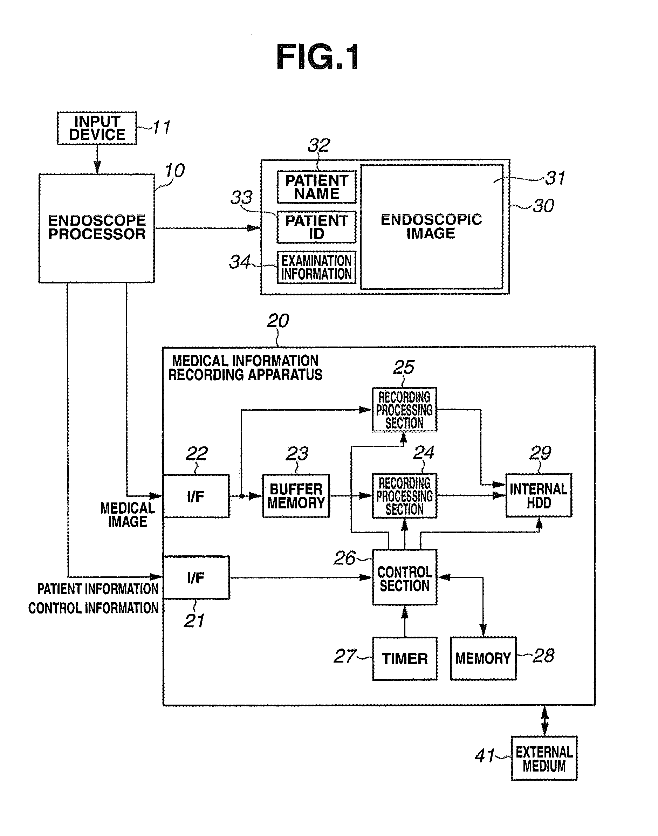

Fig. 1 is a block diagram illustrating a medical information recording system in which a medical information recording apparatus according to a first embodiment of the present invention is incorporated.

The present embodiment will be described in terms of an example where an endoscope processor 10 is used as an apparatus that outputs medical information. The endoscope processor 10 can load an image from, e.g., a non-illustrated endoscope therein and perform image signal processing to generate a medical image such as an endoscopic image. The medical image from the endoscope processor 10 is supplied to a medical information recording apparatus 20 and a monitor 30.

An input device 11 includes a non-illustrated keyboard and/or, e.g., buttons and switches (not illustrated) provided at a body of the endoscope processor 10, and enables, e.g., an operation to give an instruction for image processing in the endoscope processor 10 and/or an operation to input patient information. The endoscope processor 10 can output the patient information to the medical information recording apparatus 20.

Also, the endoscope processor 10 generates control information for controlling the medical information recording apparatus 20. Examples of the control information include, e.g., image recording start information for ordering a start of recording a medical image that is a moving image, image recording end information for ordering an end of the image recording, a still image recording information for ordering recording of a medical image that is a still image, and examination end information for ordering an end of an examination. For example, the endoscope processor 10 is configured so that image processing is controlled in response to various types of scope operations of, e.g., a release button and/or an image recording start button provided at the endoscope and the endoscope processor 10 can generate control information according to the various types of scope operations and output the control information to the medical information recording apparatus 20.

Furthermore, the endoscope processor 10 can superimpose patient information on a medical image generated based on an output of, e.g., the endoscope, and output the medical image. In such case, the endoscope processor 10 can also generate a medical image including a display region based on the patient information and a display region based on the endoscopic image.

The monitor 30 can display a medical image from the endoscope processor 10 on a screen. In the example in Fig. 1, an endoscopic image, a patient name, patient ID and examination information are displayed in a region 31 on the right side and regions 32 to 34 on the left side of the screen of the monitor 30, respectively.

The medical information recording apparatus 20 includes an interface (hereinafter referred to as I/F) 21 via which information is provided/received to/from the endoscope processor 10 and an I/F 22 via which a medical image from the endoscope processor 10 is loaded therein. The I/F 21 is, for example, an interface based on the RS-232C standard, and loads patient information and control information from the endoscope processor 10. The I/F 22 is an interface suitable for image transmission, and loads a medical image from the endoscope processor 10.

When control information generated in response to an operation of the image recording start button and an image recording end button provided at the endoscope is provided via the I/F 21, the medical information storage apparatus 20 starts recording of a medical image that is a moving image loaded via the I/F 21 (hereinafter also referred to as original moving image) and ends the recording.

In the present embodiment, when, for example, the release button provided at the endoscope is operated during recording of a medical image (original moving image), the medical information recording apparatus 20, based on control information provided by the endoscope processor 10 in response to the operation (hereinafter referred to as partial moving image recording control information), records a part of an original moving image for a period according to a timing at which the partial moving image recording control information is provided as a partial moving image separately from the original moving image, that is, generates a copy.

In order to record partial moving images, a medical image loaded from the I/F 22 is supplied to a buffer memory 23. The buffer memory 23 successively accumulates the medical image for a predetermined period and outputs the medical image to a recording processing section 24. The medical image loaded from the I/F 22 is directly provided also to a recording processing section 25. The recording processing sections 24 and 25 each have a same configuration, and the recording processing section 25 performs recording processing for original moving image recording under the control of a control section 26, and the recording processing section 24 performs recording processing for partial moving image recording under the control of the control section 26. For example, the recording processing sections 24 and 25 perform encoding processing on an inputted medical image according to a predetermined encoding method such as H.264 to compress an original moving image or a partial moving image and then supply the compressed image to an internal HDD 29. The internal HDD 29 converts the original moving image from the recording processing section 25 and the partial moving image from the recording processing section 24 into respective files and records the files therein under the control of the control section 26.

Note that the control section 26 can provide the original moving image and the partial moving image recorded in the internal HDD 29 to an external medium 41 to record the original moving image and the partial moving image in the external medium 41. Note that examples of the external medium include various types of recording media such as external hard disks and semiconductor recording media.

The patient information and the control information loaded via the I/F 21 are provided to the control section 26. The control section 26 causes the patient information and the control information to be stored in a memory 28 and controls recording processing for the medical image based on the control information.

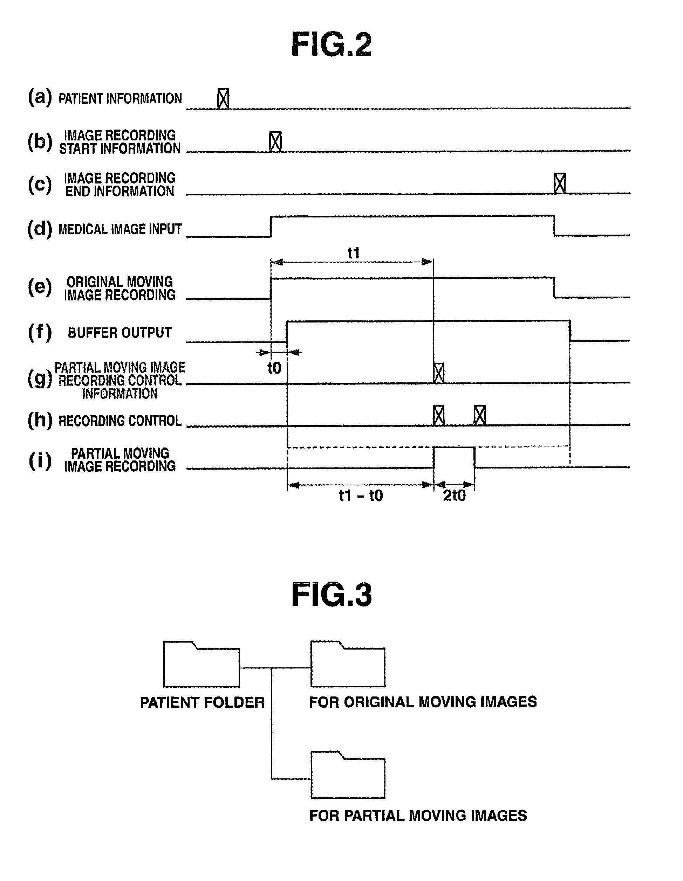

Fig. 2 is a timing chart for describing recording processing for a medical image in the present embodiment: Fig. 2(a) indicates patient information; Fig. 2(b) indicates image recording start information; Fig. 2(c) indicates image recording end information; Fig. 2(d) indicates a medical image input; Fig. 2(e) indicates recording of an original moving image; Fig. 2(f) indicates an output of the buffer memory 23; Fig. 2(g) indicates partial moving image recording control information; Fig. 2(h) indicates recording control for partial moving image recording; and Fig. 2(i) indicates recording of a partial moving image.

Upon input of patient information, which is indicated in Fig. 2(a), via the I/F 21, the control section 26 provides the patient information to the memory 24 to store the patient information in the memory 24. Each time patient information is inputted, the control section 26 creates a folder for the relevant patient information in the internal HDD 29.

Fig. 3 is a diagram for describing a folder created for each piece of patient information. The example in Fig. 3 indicates that a patient folder for each patient is created and furthermore a folder for storing original moving images and a folder for storing partial moving images are created as subfolders of the patient folder.

Upon input of image recording start information, which is indicated in Fig. 2(b), via the I/F 21, the control section 26 starts recording of an original moving image (Fig. 2(e)). A medical image from the endoscope processor 10 is loaded to the medical information recording apparatus 20 via the I/F 22, and supplied to the recording processing section 25 directly from the I/F 22 (Fig. 2(d)). When image recording start information is provided, the recording processing section 25 immediately starts image recording as indicated in Fig. 2(e). In other words, the recording processing section 25 performs proper processing such as compression processing on the medical image inputted via the I/F 22 and then provides the medical image to the internal HDD 29 to record the medical image in the internal HDD 29. Note that although the present embodiment is described in terms of an example in which, e.g., a medical image is recorded in the internal HDD 29, it is clear that the external medium 41 such as an external HDD and/or any of various types of recording media can be selected as a destination of recording of, e.g., a medical image.

The recording of the original moving image, which is a moving medical image, is continued until image recording end information, which is indicated in Fig. 2(c), is inputted. Upon input of image recording end information, the control section 26 controls the recording processing section 25 to terminate the recording of the medical image. The recording processing section 25 converts the original moving image into a file and the file is recorded in a subfolder for original moving images in a patient folder created in the internal HDD 29, with a proper file name provided thereto.

As described above, in the present embodiment, the control section 26 and the recording processing section 25 causes a medical image (original moving image) for a moving image recording period designated by image recording start information and image recording end information to be recorded as one image file in the internal HDD 29.

Here, it is assumed that during a moving image recording period for an original moving image, a surgeon operates, for example, a release button provided at the non-illustrated endoscope. The endoscope processor 10 generates partial moving image recording control information according to the operation of the release button and outputs the partial moving image recording control information to the medical information recording apparatus 20 (Fig. 2(g)). The moving image recording control information is provided to the control section 26 via the I/F 21. When the partial moving image recording control information is provided, the control section 26 instructs the recording processing section 24 to record a medical image. Also, the control section 26 sets an image recording period for the recording processing section 24 to a predetermined period, and when the image recording period ends, terminates recording by the recording processing section 24. The recording processing section 24 performs recording of a medical image, that is, a part of an original moving image according to such control of the control section 26.

A medical image is supplied to the recording processing section 24 via the buffer memory 23. As indicated in Fig. 2(f), the buffer memory 23 outputs the medical image inputted via the I/F 22 while storing the medical image for a predetermined period t0. Accordingly, the medical image supplied to the recording processing section 25 is supplied to the recording processing section 24 with a delay of the time period t0. If it is assumed that partial moving image recording control information is generated at a timing a time period t1 after recording of a head of the medical image by the recording processing section 25, the recording processing section 24 starts recording from the medical image a time period of (t1-t0) after the start of recording of the original moving image as indicated in Fig. 2(i). In other words, where, e.g., a delay in transmission of control information and a delay in recording processing are ignored, in the recording processing section 24, recording is started from the medical image at a timing the time period t0 before the timing at which the surgeon operates the release button.

Also, where the control section 26 sets the image recording period for the recording processing section 24 to 2t0 (Fig. 2(i)), the medical image is recorded until a timing the time period t0 after the timing at which the surgeon operates the release button. For example, if the delay time period t0 for the buffer memory 23 is set to two minutes, the image can be recorded for four minutes as a result of totaling two minutes before and two minutes after the operation of the release button by the surgeon.

Upon end of the image recording period, the recording processing section 24 converts the partial moving image into a file and records the file in the subfolder for partial moving images in the patient folder (examination folder) created in the internal HDD 29, with a proper file name provided thereto.

As described above, in the present embodiment, the control section 26 and the recording processing section 24 record a partial moving image for a predetermined time period before and after an operation of the release button in an original moving image as one image file in the internal HDD 29.

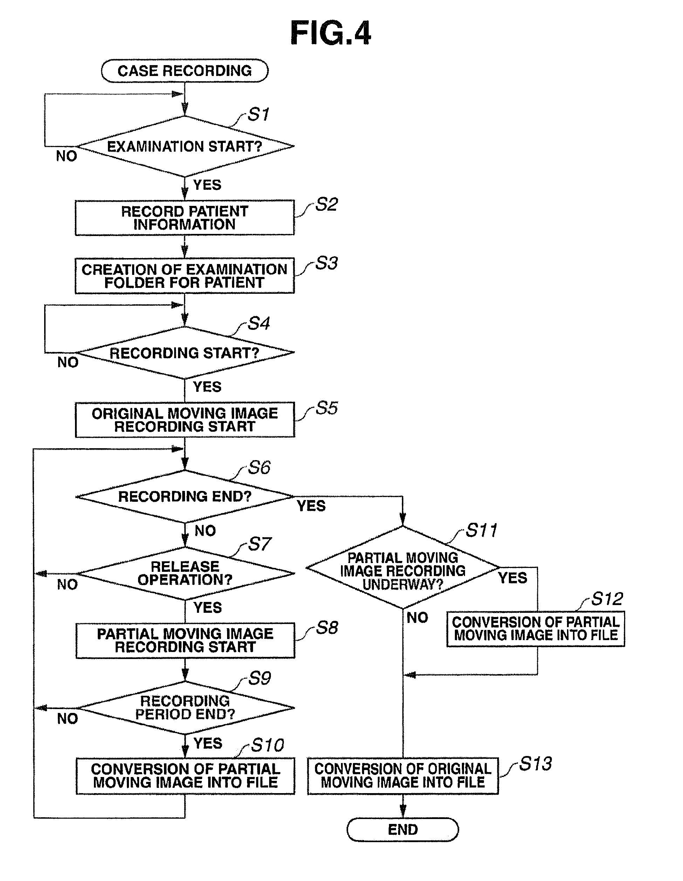

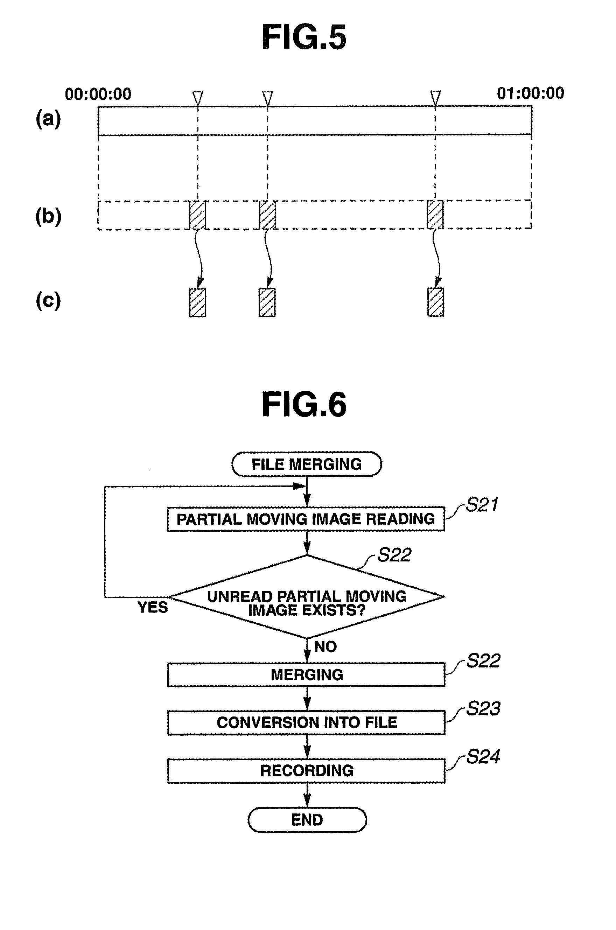

Next, an operation of the embodiment configured as described above will be described with reference to the flowchart in Fig. 4 and the diagram in Fig. 5. Fig. 5(a) indicates an original moving image; Fig. 5(b) indicates a correspondence between the original moving image and partial moving images (shaded parts); and Fig. 5(c) indicates the partial moving images.

In step S1 in Fig. 4, the control section 26 determines whether or not an examination is started. For example, the control section 26 determines that an examination is started, by an operation such as an input of patient information, an image recording start operation or an examination start operation. A surgeon operates the input device 11 to input patient information. The patient information is supplied from the endoscope processor 10 to the medical information recording apparatus 20. The patient information is loaded to the control section 26 via the I/F 21 and recorded in the memory 28 (step S2).

The control section 26 analyzes the inputted patient information and creates an examination folder with a folder name based on the patient information in the internal HDD 29 (step S3). For example, the control section 26 creates an examination folder with a folder name including patient ID. Also, the control section 26 creates a subfolder for original moving images and a subfolder for partial moving images in the folder.

It is assumed that the surgeon presses the image recording start button provided at, e.g., the non-illustrated endoscope or the endoscope processor 10 to order a start of image recording. In response to the operation, the endoscope processor 10 outputs, for example, a medical image generated by superimposing the patient information on an endoscopic image from the endoscope to the medical information recording apparatus 20, and generates image recording start information and outputs the image recording start information to the medical information recording apparatus 20. Upon receipt of the image recording start information via I/F 21 (step S4), the control section 26 loads the medical image inputted via the I/F 22. The medical image is provided to the recording processing section 25 as an original moving image, and supplied to the internal HDD 29 as a result of recording processing in the recording processing section 25, whereby recording is started (step S5).

In next step S6, an end of the image recording is determined. When the surgeon presses the image recording end button, in step S 11, whether or not recording of a partial moving image is underway is determined, and if no partial moving image is being recorded, in step S 13, recording processing for the original moving image is ended and the original moving image is converted into a file. The original moving image file is recorded in the subfolder for an original moving image in the patient folder.

Fig. 5(a) indicates an original moving image recorded as described above, and the example in Fig. 5 indicates that a medical image has been recorded for one hour from 00:00:00 to 01:00:00 as an original moving image.

Next, it is assumed that the release button provided at, e.g., the endoscope or the endoscope processor 10 is pressed during the original moving image being recorded. The triangle marks in Fig. 5(a) each indicate a timing of an operation of the release button, and in the example in Fig. 5, the release button is operated three times during a period in which the original moving image is being recorded. When the release button is pressed, the endoscope processor 10 generates partial moving image recording control information and outputs the partial moving image recording control information to the medical information recording apparatus 20. Upon receipt of the moving image recording control information via the I/F 21, the control section 26 determines that a start of recording a partial moving image is ordered by a release operation (step S7), and makes the recording processing section 24 start to record a medical image inputted to the recording processing section 24 from I/F 22 via the buffer memory 23 (step S8).

The medical image inputted to the recording processing section 24 is one with a delay caused by the buffer memory 23 relative to the medical image inputted to the recording processing section 25. Accordingly, the medical image recorded by the recording processing section 24 is an image at a timing temporally before the medical image recorded by the recording processing section 25. Accordingly, where a delay of recording of the original moving image relative to the surgeon's recording operation is ignored, recording of the medical image as a partial moving image is started from a time before the surgeon's release operation by the delay time in the buffer memory 23.

After the elapse of a predetermined period, the control section 26 determines in step S9 that the partial moving image recording period has ended, and makes the recording processing section 24 terminate the image recording. Consequently, the partial moving image is converted into a file and the file is recorded in the subfolder for partial moving images in the patient folder (step S10). As described above, as indicated in the shaded parts in Fig. 5(b), a medical image for a predetermined period before and after each of release button operation timings is recorded as a partial moving image.

Note that if the image recording end button is pressed by the surgeon during the recording of a partial moving image, the processing moves from step S11 to step S12 and the recording of the partial moving image is terminated and the partial moving image is converted into a file and the file is recorded in the internal HDD 29, and then the original moving image is converted into a file and the file is recorded. In other words, as indicated in Fig. 5(c), each of a number of partial moving images, the number corresponding to the number of release button operations, is converted into a file and the file is recorded independently from the original moving image.

For example, during an examination, a surgeon may recognize a part as especially important in a medical image that is being recorded. In such case, the surgeon can record a partial moving image that he/she considers important separately from the original moving image by means of a simple operation to operate, e.g., the release button. In this case, the partial moving image can include the medical image at a timing a predetermined period before the surgeon's operation of, e.g., the release button, and thus, it is highly likely that the part that the surgeon considers necessary can reliably be recorded as a partial moving image.

At the time of reproduction, for example, the partial moving images are reproduced, whereby, e.g., a point in the examination and the flow of the examination can be understood in a short period of time.

As described above, in the present embodiment, a simple operation for recording a partial moving image is performed at a point during recording of a medical image, enabling recording of the medical image before and after the operation separately from the original moving image. Consequently, only a desired part can easily be recorded from a medical image that has been recorded for a relatively long period of time and the desired part can easily be reproduced.

In particular, a merit of enabling recording of a part of a moving image before a release that is a starting point lies in that a process until an optimum endoscope image is displayed and/or how to use forceps or a treatment instrument for a lesional site before a treatment can be recorded. For example, a release is performed when a lesional site is diagnosed, whereby how to approach the scope to the lesional site can be understood, which serves as a useful educational material for young doctors. Likewise, a release is performed when a treatment is about to be performed or during a treatment, whereby a how to treat a lesional site and/or how to approach a treatment instrument to the lesional site can be understood. In addition, a release is performed after a treatment, whereby, for example, a hemostatic treatment and/or a procedure for a hemostasis check and an accompanying check of the condition of the patient can be recorded. When image recording is terminated, the surgeon can promptly reproduce and check correctness of the treatment at the surgery site, and the recorded data serves as a useful educational material for young doctors.

Possibility of realization of the recording method according to the present embodiment enables reduction in burden on doctors that perform moving image editing and contribution to enhancement in medical technique when recorded data is utilized.

Note that a timing for starting recording a partial moving image in a medical image can arbitrarily be changed by changing the delay time in the buffer memory 23. Furthermore, a timing for terminating the recording of the partial moving image in the medical image can arbitrarily be changed by a setting made by the control section. Furthermore, the recording of the partial moving image may be terminated by a second release button operation. Furthermore, although an example in which recording of a partial moving image is performed in response to a surgeon's release button operation has been described, an operation that serves as a trigger for recording a partial moving image can arbitrarily be changed.

### (Second Embodiment)

Fig. 6 is a flowchart illustrating an operation flow employed in a second embodiment of the present invention. A hardware configuration in the present embodiment is similar to that of the first embodiment, and is different from that of the first embodiment only in recording control in the control section 26.

The first embodiment has been described in terms of an example in which file(s) of one or more partial moving images are recorded in response to operation(s) of, e.g., a release button. In the present embodiment, when files of a plurality of partial moving images are formed from one original moving image, the files of the partial moving images are merged to create one partial moving image file.

In the present embodiment, processing until partial moving images are generated is similar to that of the first embodiment. Upon end of conversion of an original moving image into a file and recording of the file in Fig. 4, the control section 26 performs processing in step S21 in Fig. 6. In step S21, the control section 26 reads partial moving images stored in a folder for partial moving images in a patient folder in the order of recording. The control section 26 reads all of partial moving images while determining whether there is no longer any unread partial moving image in step S22.

When all of partial moving images stored in the folder for partial moving images are read, the control section 26 merges the partial moving images in the order of recording (step S23). Note that the processing for reading and merging the partial moving images in the control section 26 is performed with the partial moving images remained compressed.

The control section 26 converts the partial moving images merged in step S23 into a file and records the file in the folder from which the partial moving images have been read, with a proper file name provided thereto (step S24).

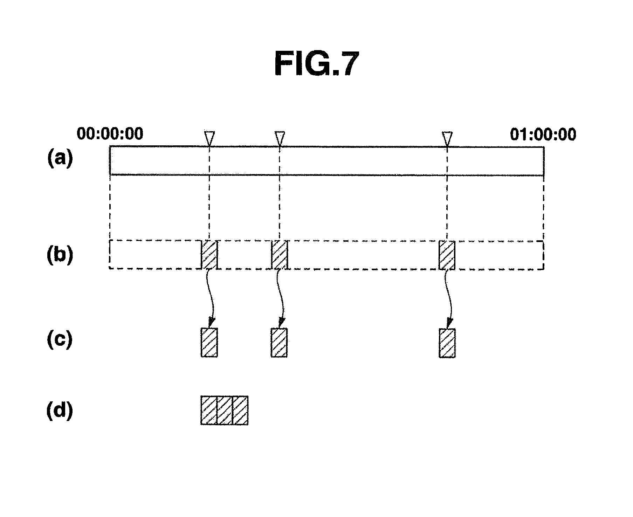

An operation of the embodiment configured as described above will be described with reference to Fig. 7. Fig. 7 is a diagram for describing a recording method in the second embodiment: Fig. 7(a) indicates an original moving image; Fig. 7(b) indicates a correspondence between the original moving image and partial moving images (shaded parts); Fig. 7(c) indicates the plurality of partial moving images; and Fig. 7(c) indicates the partial moving images after merging.

In the present embodiment, as in the first embodiment, when a release operation occurs in a period in which an original moving image is being recorded, which is illustrated in Fig. 7(a), at each of timings of release operations indicated by the triangle marks in Fig. 7(a), a partial moving image is created (Fig. 7(b)). As illustrated in Fig. 7(c), the partial moving image is converted into a file and the file is recorded.

Furthermore, in the present embodiment, the control section 26 reads and merges the partial moving images to generate a partial moving image, which is indicated in Fig. 7(d). The partial moving image resulting from the merging is also stored in the folder for partial moving images.

As described above, in the present embodiment, a plurality of partial moving images are merged to generate one partial moving image, and thus, when a plurality of partial moving images are generated, at the time of reproduction of the partial moving images, the reproduction can easily be performed.

Note that although the present embodiment has been described in terms of an example in which a plurality of partial moving images are merged after conversion of an original moving image into a file and recording of the file, a timing for merging the partial moving images can arbitrarily be set, and for example, each time a partial moving image is created, the created partial moving image may be merged to the last of the previous partial moving images, or when a predetermined number of partial moving images are created, the partial moving images may be merged.

Furthermore, although the present embodiment has been described in terms of an example in which when a plurality of partial moving images are created, the partial moving images are unconditionally merged, whether or not the partial moving images are merged may be determined according to an operation by a user.

### (Third Embodiment)

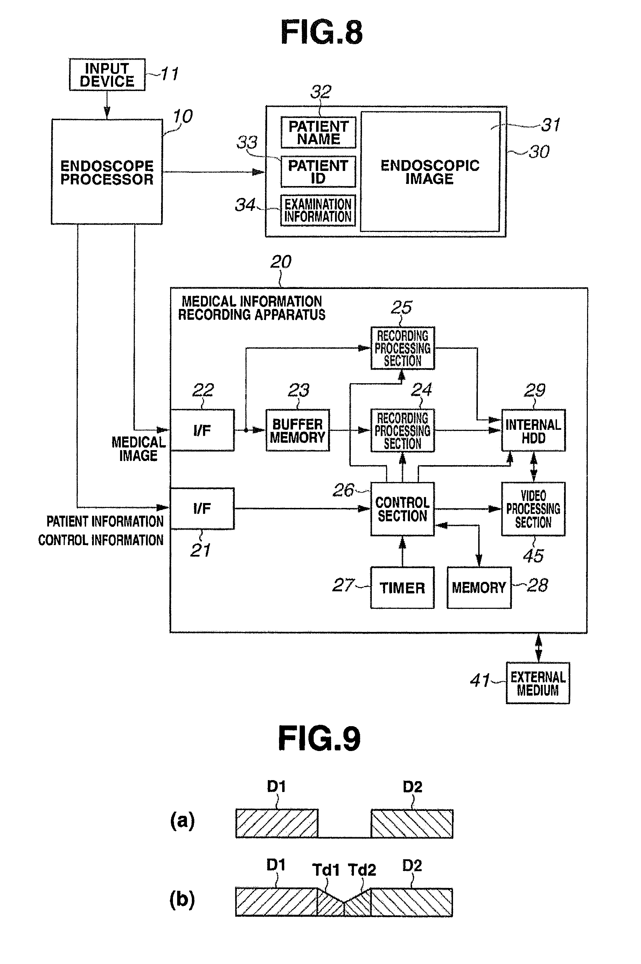

Fig. 8 is a block diagram illustrating a third embodiment of the present invention. In Fig. 8, components that are the same as those in Fig. 1 are provided with reference numerals that are the same as those in Fig. 1, and a description thereof will be omitted.

The present embodiment is different from the second embodiment in that a video processing section 45 is added. In the second embodiment, the control section 26 simply merges a plurality of partial moving images in chronological order. On the other hand, in the present embodiment, transient images are inserted in merging of partial moving images.

Partial moving images to be merged correspond to regions of a medical image that are different in time from each other, and thus, can be considered as having no continuity in image. Therefore, it is probable that when a partial moving image resulting from merging is reproduced, the image noticeably changes at merging parts, providing a moving image that is difficult to see. Therefore, transient images for making it easy to see the merging parts are inserted between the merging parts.

The video processing section 45 performs insertion of such transient images under the control of the control section 26. In other words, the video processing section 45 reads partial moving images from the internal HDD 29 under the control of the control section 26. The video processing section 45 decodes the read partial moving images to analyze parts of images corresponding to a predetermined period before and after merging parts. The video processing section 45 generates transient images based on the image analysis for merging parts of partial moving images to be merged.

Fig. 9 is a diagram for describing an example of a method for generating transient images. The video processing section 45 reads partial moving images D1 and D2, which are illustrated in Fig. 9(a), and merges a head of the partial moving image D2 to an end of the partial moving image D1. In this case, the video processing section 45 generates a transient image Td1 resulting from a luminance of a last image in the partial moving image D1 being gradually lowered, and a transient image Td2 resulting from a luminance of the head image in the partial moving image D2 being gradually returned to an initial luminance from a state in which the luminance of the head image is lowered. Then, as illustrated in Fig. 9(b), the video processing section 45 merges the partial moving image D1, the transient images Td1 and Td2 and the partial moving image D2.

Note that the video processing section 45 can create transient images by means of various methods, which are not limited to the method in Fig. 9. For example, the video processing section 45 may generate a transient image resulting from a luminance of the image for a predetermined last period of time in the partial moving image D1 being gradually lowered, and likewise, may generate a transient image resulting from a luminance of the image for a predetermined period of time in the head of the partial moving image D2 being gradually returned to an initial luminance from a state in which the luminance is lowered. Furthermore, for example, if a difference in luminance between the partial moving images D1 and D2 is large, one transient image with a brightness changing from the luminance of the last image in the partial moving image D1 to the luminance of the head image in the partial moving image D2 may be generated to insert the generated transient image between the partial moving images D1 and D2.

The video processing section 45 encodes the partial moving image resulting from the merging according to a predetermined encoding method such as H.264, and then provides the partial moving image to the internal HDD 29 to store the partial moving image in a folder for partial moving images.

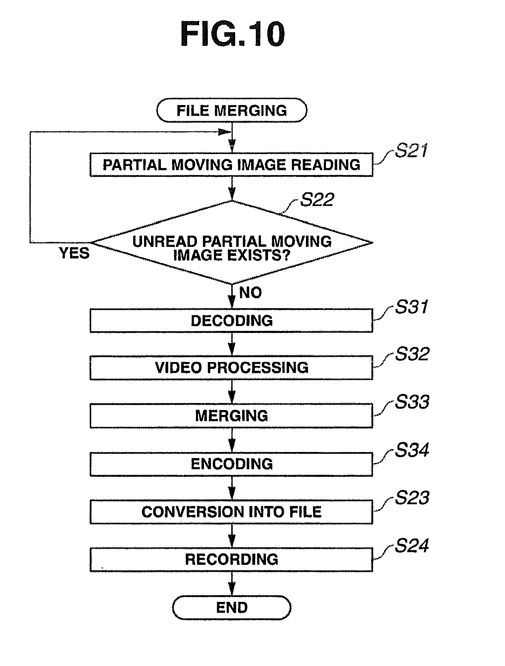

Next, an operation of the embodiment configured as described above will be described with reference to Fig. 10. Fig. 10 is a flowchart illustrating an operation of the third embodiment. In Fig. 10, steps that are the same as those in Fig. 6 are provided with reference numerals that are the same as those in Fig. 6, and a description thereof will be omitted.

The video processing section 45 reads partial moving images stored in the internal HDD 29, under the control of the control section 26 (steps S21 and S22). The video processing section 45 decodes the partial moving images read in step S31 and generates transient images by means of video processing (step S32). The video processing section 45 merges the partial moving images while inserting the transient images between the partial moving images.

The video processing section 45 encodes a partial moving image resulting from the merging (step S34). The video processing section 45 converts the encoded partial moving image into a file and records the file in the internal HDD 29 (steps S23 and S24).

As described above, in the present embodiment, partial moving images are merged with transient images inserted therebetween, providing the advantage of making it easy to see an image at a joint when a partial moving image resulting from the merging is reproduced.

### (Fourth Embodiment)

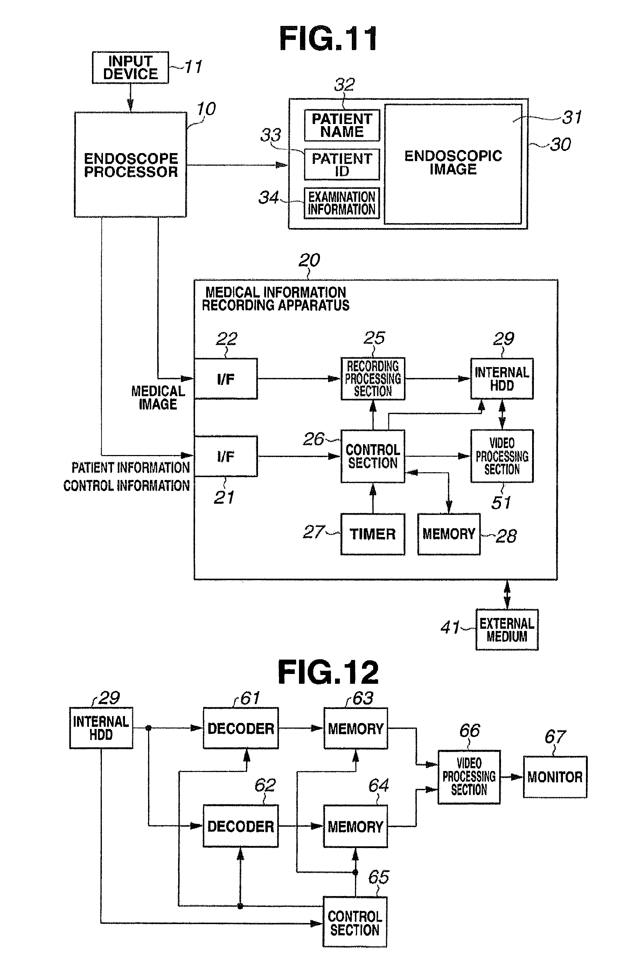

Fig. 11 is a block diagram illustrating a fourth embodiment of the present invention. In Fig. 11, components that are the same as those in Fig. 1 are provided with reference numerals that are the same as those in Fig. 1, and a description thereof will be omitted. The present embodiment is different in configuration from the first embodiment in that the buffer memory 23 and the recording processing section 24 are omitted and a video processing section 51 is added.

The first embodiment has been described in terms of an example in which partial moving images are recorded simultaneously with recording of an original moving image in an internal HDD 29. On the other hand, in the present embodiment, a partial moving image is generated after recording of an original moving image. A recording processing section 25 records a medical image inputted via an I/F 22 as an original moving image in an internal HDD 29, under the control of a control section 26.

In the present embodiment, the control section 26 records control information, especially partial moving image recording control information, which is inputted via the I/F 21, in association with a time for an original moving image, using time information from a timer 27. Upon end of recording of the original moving image, the control section 26 instructs the video processing section 51 to create a partial moving image.

The video processing section 51 decodes and reproduces the original moving image. The partial moving image recording control information is provided to the video processing section 51 from the control section 26. The video processing section 51 copies a predetermined region of the original moving image based on the partial moving image recording control information to provide a partial moving image. In other words, the video processing section 51 determines a predetermined period before and after a position based on partial moving image recording control information as a region to be copied.

As described above, partial moving image recording control information is generated based on, e.g., a surgeon's release operation, and the video processing section 51 can copy a moving image that is a part corresponding to a predetermined period before and after the surgeon's release operation from the original moving image as a partial moving image. The video processing section 51 stores the copied partial moving image in a folder for partial moving images in the internal HDD 29.

As described above, in the present embodiment, as in the first embodiment, a simple operation for recording a partial moving image is performed at a point during recording of a medical image, enabling a medical image before and after the operation to be recorded separately from the original moving image.

Note that although the present embodiment has been described in terms of an example in which the first embodiment is applied, it is clear that the second or third embodiment may be applied to the present embodiment to merge a plurality of partial moving images.

### (Reproduction Apparatus)

Fig. 12 is a block diagram illustrating an example of a reproduction apparatus.

At the time of recording a medical image, chapters (marks) may be set automatically or according to a surgeon's operations. Information on such chapters is recorded in an internal HDD 29 in a recording apparatus in association with the medical image. At the time of reproduction, a reproduction position in the medical image can be jumped to a position of a timing corresponding to the chapter (hereinafter chapter point) by referring to the chapter information.

In this case, a reproduction apparatus, which is illustrated in Fig. 12, enables image parts corresponding to consecutive chapter points in one medical image to be simultaneously reproduced.

A medical image from the internal HDD 29 is supplied to decoders 61 and 62. The decoder 61 is one for main reproduction, and the decoder 62 is one for sub-reproduction. The decoders 61 and 62 decode the inputted medical image and output the medical image to memories 63 and 64, respectively, under the control of a control section 65.

The control section 65 reads chapter information from the internal HDD 29. The control section 65 controls the decoders 61 and 62 so that a reproduction point in the medical image is moved to a chapter point based on an operation by a user. In this case, the control section 65 designates, for example, a chapter point following a chapter point designated as a reproduction position for the decoder 61, as a reproduction position for the decoder 62.

Also, the control section 65 controls writing and reading to and from the memories 63 and 64, thereby reading the medical image for main reproduction from the memory 63 and the medical image for sub-reproduction from the memory 64. A video processing section 66 performs video processing for simultaneously displaying the medical image for main reproduction and the medical image for sub-reproduction on one screen and outputs the medical images to a monitor 67.

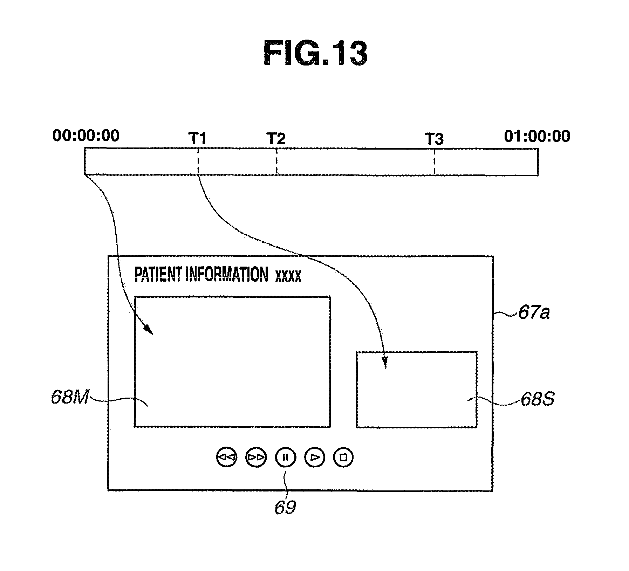

Next, an operation of the reproduction apparatus configured as described above will be described with reference to the diagram in Fig. 13.

Now, it is assumed that a medical image for one hour from a time 00:00:00 to a time 01:00:00 is recorded in the internal HDD 29. Fig. 13(a) indicates such medical image, and in the medical image, three chapters T1, T2 and T3 are set.

At the time of reproduction, the control section 65 reads chapter information. A user orders reproduction of the medical image in Fig. 13(a). The control section 65 controls the decoders 61 and 62 to read the medical image from the internal HDD 29. In this case, the control section 65 reproduces medical data at reproduction positions that are different for main reproduction and sub-reproduction. In other words, the control section 65 instruct the decoder 61 to reproduce the medical image from a start position and instructs the decoder 62 to reproduce the medical image from the first chapter T1.

The decoders 61 and 62 read the medical image from the internal HDD 29 and decode the medical image, and output the medical images for the respective positions instructed by the control section 65 to the memories 63 and 64, respectively. The control section 65 controls writing and reading to and from the memories 63 and 64 to read the medical image for main reproduction from the memory 63 and read the medical image for sub-reproduction from the memory 64. The video processing section 66 combines the medical images and outputs the resulting image to the monitor 67.

Fig. 13(b) illustrates a display example on a display screen 67a of the monitor 67. The example in Fig. 13(b) indicates that a display region 68M for main reproduction is provided on the left side of the display screen 67a and a display region 68S for sub-reproduction is provided on the right side, and in the display region 68M, a medical image based on an output of the decoder 61 is displayed, and in the display region 68S, a medical image based on an output of the decoder 62 is displayed simultaneously with the display in the display region 68M. In other words, in the display region 68M, a medical image from the time 00:00:00 is displayed, and in the display region 68S, a medical image from the chapter T1 is displayed.

Here, when a user orders reproduction from the chapter T1, the control section 65 controls the decoder 61 so as to change a reproduction position for the decoder 61 to the chapter T1, and controls the decoder 62 so as to change a reproduction position for the decoder 62 to the chapter T2. Consequently, in the display region 68M, the medical image from the chapter T1 is displayed, and in the display region 68S, the medical image from the chapter T2 is displayed.

The reproduction apparatus in Fig. 12 enables medical images in two consecutive chapters to be simultaneously reproduced.

Furthermore, on the display screen 67a, various types of button displays 69 for controlling medical image reproduction are displayed. The user's operation of the button display 69 enables reproduction, a reproduction stop, a pause, a fast-forward or a rewind of a medical image. The control section 65 simultaneously controls reproduction of both medical images for main reproduction and sub-reproduction based on an operation of the button display 69. For example, if an operation for fast-forward of a medical image for main reproduction is performed, in the display region 68M, the medical image for main reproduction is reproduced by fast-forwarding, and simultaneously, in the display region 68S, the medical image for sub-reproduction is also reproduced by fast-forwarding.

As described above, the reproduction apparatus in Fig. 12 enables medical images in two consecutive chapters to be simultaneously reproduced, and thus a user can easily recognize a desired reproduction position in a medical image.

Note that although the apparatus in Fig. 12 simultaneously reproduces medical images shifted from each other by one chapter, a difference (chapter count) between reproduction positions for simultaneous reproduction can arbitrarily be set.

### (Fifth Embodiment)

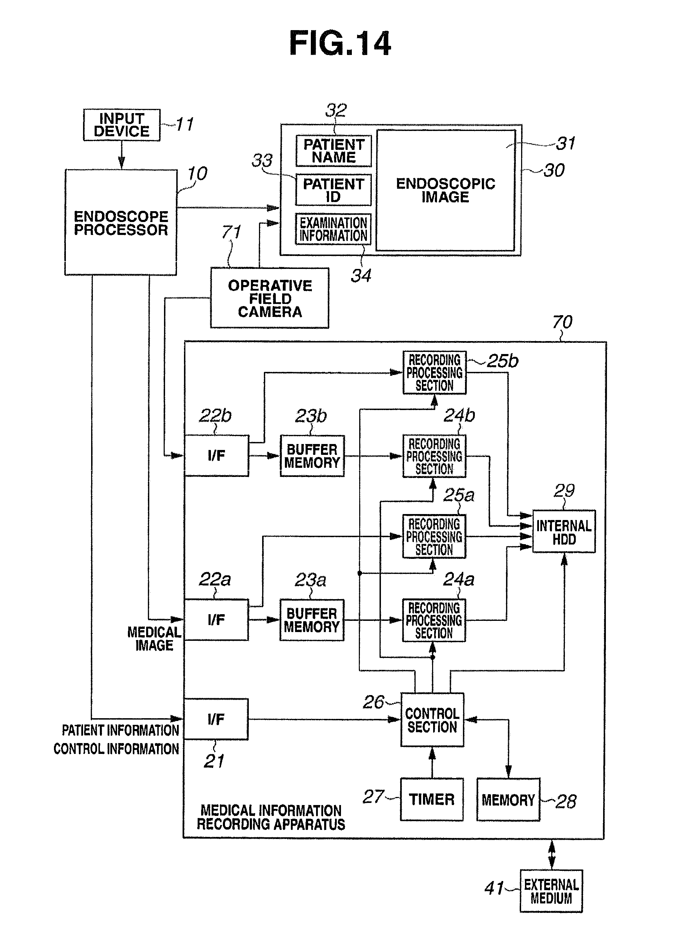

Fig. 14 is a block diagram illustrating a fifth embodiment of the present invention. In Fig. 14, components that are the same as those in Fig. 1 are provided with reference numerals that are the same as those in Fig. 1, and a description thereof will be omitted.

The present embodiment is one employing not only an endoscope processor 10 but also an operative field camera 71 as an apparatus that outputs medical information. The operative field camera 71 picks up an image of an operative field and supplies the picked-up image to a medical information recording apparatus 70 and a monitor 30 as a medical image (hereinafter also referred to as operative field image). Consequently, the monitor 30 can display not only a medical image from the endoscope processor 10 but also a medical image from the operative field camera 71 on a screen. The monitor 30 also can display an endoscopic image and an operative field image simultaneously.

The medical information recording apparatus 70 in the present embodiment includes two processing sections that are each similar to the medical information recording apparatus 20 in Fig. 1. In other words, each of I/Fs 22a and 22b, each of buffer memories 23a and 23b, each of recording processing sections 24a and 24b and each of recording processing sections 25a and 25b have configurations that are the same as those of the I/F 22, the buffer memory 23, the recording processing section 24 and the recording processing section 25, respectively.

The I/Fs 22a and 22b are interfaces suitable for image transmission, and load a medical image from the endoscope processor 10 and a medical image from the operative field camera 71, respectively. Note that if one I/F 22 includes two input terminals (not illustrated), a plurality of medical images may be loaded by the one I/F 22 only. For example, plural types of terminals such as a DVI (digital visual interface) terminal, an SDI (serial digital interface) terminal, an RGB terminal, a Y/C terminal and a VIDEO terminal can be employed for input terminals. Any of an ultrasonic apparatus, an X-ray observation apparatus and an endoscope processor other than the endoscope processor 10, other than an endoscope processor and/or an operative field camera, can be connected to the input terminals.

The I/F 22a supplies a medical image from the endoscope processor 10 to the buffer memory 23a and the recording processing section 25a, and the I/F 22b supplies a medical image from the operative field camera 71 to the buffer memory 23b and the recording processing section 25b. The buffer memories 23a and 23b sequentially accumulate respective medical images for a predetermined period, and outputs the respective accumulated images to the recording processing sections 24a and 24b, respectively.

In the present embodiment, as in the first embodiment, the recording processing sections 25a and 25b performs recording processing for original moving image recording under the control of the control section 26, and the recording processing sections 24a and 24b performs recording processing for partial moving image recording under the control of the control section 26. The rest of the configuration is similar to that of the first embodiment, and the present embodiment is different from the first embodiment in that an operative field image from the operative field camera 71 is recorded simultaneously with an endoscopic image as an original moving image and an endoscopic image and an operative field image from the operative field camera 71 are recorded as partial moving images.

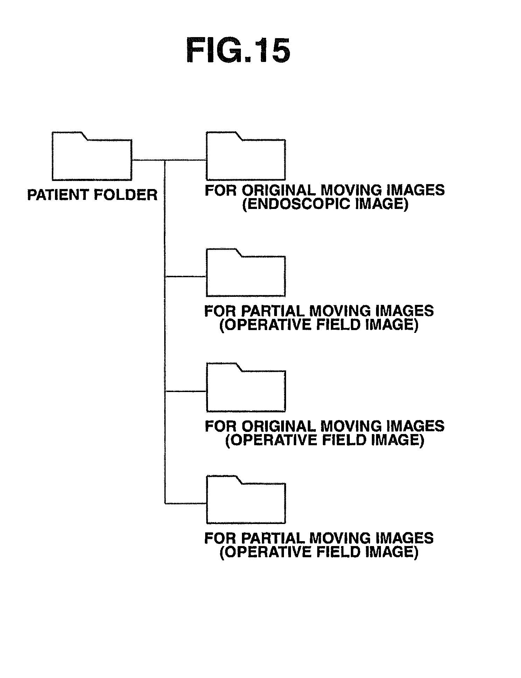

In the embodiment configured as described above, as in the first embodiment, recording of an original moving image and a partial moving image is performed. In other words, when an examination is started, the control section 26 analyzes inputted patient information and creates an examination folder with a folder name based on the patient information in an internal HDD 29. For example, the control section 26 creates an examination folder with a folder name including patient ID. Also, the control section 26 creates a subfolder for original moving images and a subfolder for partial moving images in the folder. In the present embodiment, as illustrated in the diagram in Fig. 15, a patient folder for each patient is created, and furthermore, not only a folder for endoscopic images in which original moving images are stored and a folder for endoscope images in which partial moving images are stored, but also a folder for operative field images in which original moving images are stored and a folder for operative field images in which partial moving images are stored are created as subfolders of the patient folder.

It is assumed that a surgeon presses an image recording start button provided at a non-illustrated endoscope or the endoscope processor 10 and the operative field camera 71 to order a start of recording. In response to this operation, the endoscope processor 10 outputs, for example, a medical image generated by superimposing patient information on an endoscopic image from the endoscope to the medical information recording apparatus 70, and generates image recording start information and outputs the image recording start information to the medical information recording apparatus 70. Also, the operative field camera 71 outputs an operative field image to the medical information recording apparatus 70. Upon receipt of the image recording start information via the I/F 21 a, the control section 26 loads two medical images inputted via the I/Fs 22a and 22b. The endoscopic image and the operative field image inputted via the I/Fs 22a and 22b, respectively, are provided to the respective recording processing sections 25a and 25b as original moving images, and also supplied to the respective recording processing sections 24a and 24b via the respective buffer memories 23a and 23b as partial moving images.

As a result of recording processing in the recording processing sections 25a and 25b, the original moving image of the endoscopic image and the original moving image of the operative field image are supplied to the internal HDD 29 and recording of the original moving images is started. When the surgeon presses an image recording end button, the image recording processing for the original moving images is terminated and the original moving images are converted into files. The original moving image files are recorded in the subfolder for original moving images (endoscope images) or the subfolder for original moving images (operative field images) in the patient folder.

Next, it is assumed that a release button provided at, e.g., the endoscope or the endoscope processor 10 is pressed during recording of original moving images. When the release button is pressed, the endoscope processor 10 generates partial moving image recording control information and outputs the partial moving image recording control information to the medical information recording apparatus 20. Upon receipt of the partial moving image recording control information via the I/F 21, the control section 26 determines that a start of partial moving image recording is ordered by the release operation, and makes the recording processing sections 24a and 24b start recording of an endoscopic image and an operative field image inputted from the I/Fs 22a and 22b to the recording processing sections 24a and 24b via the buffer memories 23a and 23b, respectively.

In other words, in the present embodiment, a medical image from the endoscope processor 10 and a medical image from the operative field camera 71 can be recorded as two partial images by means of an operation of, e.g., the release button at the endoscope processor 10.

A medical image (endoscopic image) inputted to the recording processing section 24a is one with a delay caused by the buffer memory 23a relative to an endoscopic image inputted to the recording processing section 25a. Accordingly, an endoscopic image recorded by the recording processing section 24a is an image at a timing temporally before an endoscopic image recorded by the recording processing section 25a. Accordingly, where a delay of original moving image recording relative to a surgeon's operation for image recording is ignored, recording of an endoscopic image as a partial moving image is started from a time before the surgeon's release operation by the delay time in the buffer memory 23a.

Likewise, a medical image (operative field image) inputted to the recording processing section 24b is one with a delay caused by the buffer memory 23b relative to an operative field image inputted to the recording processing section 25b. Accordingly, an operative field image recorded by the recording processing section 24b is an image at a timing temporally before an operative field image recorded by the recording processing section 25b. Accordingly, where a delay of original moving image recording relative to the surgeon's operation for image recording is ignored, recording of an operative field image as a partial moving image is started from a time before the surgeon's release operation by the delay time in the buffer memory 23b.

After the elapse of a predetermined period, the control section 26 determines that a recording period for the partial moving images has ended, and makes the recording processing sections 24a and 24b terminate the image recording. Consequently, the two partial moving images are converted into files and the files are recorded in the subfolder for partial moving images (endoscopic images) and the subfolder for partial moving images (operative field images) in the patient folder, respectively. As described above, the medical images (the endoscopic image and the operative field image) for the predetermined period before and after a timing of an operation of the release button are recorded as partial moving images.

Note that if the image recording end button is pressed by the surgeon during a period in which two partial moving images are being recorded, recording of the two partial moving images is terminated and the two partial moving images are converted into files and the files are recorded in the internal HDD 29, and then two original moving images are converted into files and the files are recorded. In other words, the number of files of each of two partial moving images, the number corresponding to the number of release button operations, are formed and the files are recorded, independently from the two original moving images.

As described above, in the present embodiment, two inputted medical images can be recorded as original moving images over an entire period, and two medical images can be recorded as partial moving images for a predetermined period before and after a timing of a release button operation. A combination of an endoscope processor and an operative field camera enables movements of a surgeon's hands and an endoscopic image to be recorded in association with each other, whereby a treatment method, an approach of an treatment instrument and/or how to move the treatment instrument can comprehensively be understood from the combination of the endoscopic image and the hand movements, rather than the endoscopic image only, and thus, the combination serves as a useful educational material for further deepening doctors' understanding. In the case of such two medical images, an endoscopic image and an operative field image can be recorded as original moving images over an entire period, and an endoscopic image and an operative field image can be recorded for a predetermined period at a timing of a release button operation, and an image with an endoscopic image and an operative field image associated with each other also enables reduction in burden on doctors that perform moving image editing and contribution to enhancement in medical technique when recorded data is utilized.

Furthermore, although an example in which a partial moving image is recorded in response to an operation of the release button by a surgeon has been described, an operation that serves as a trigger for a partial moving image recording can arbitrarily be changed, and for example, a partial moving image may be recorded in response to an operation of a release button at the operative field camera.

Note that although an example in which two medical images are recorded as original moving images and also recorded as partial moving images has been described, one medical image of two medical images may be recorded as an original moving image and the other may be recorded as a partial moving image. For example, it is possible that an endoscopic image is recorded as an original moving image over an entire period while an operative field image is recorded for a predetermined period at a timing of a release button operation.

Also, it is clear that any of the second to fourth embodiments can be applied to the fifth embodiment.

The present application is filed claiming the priority of Japanese Patent Application No. 2011-120856 filed in Japan on May 30, 2011, and the above described disclosure is incorporated by reference in the present description, claims and drawings.

## Claims

1. A medical information recording apparatus comprising:
a control section that controls moving image recording of a medical image from a medical apparatus;
a first recording processing section that records the medical image in a first recording region over an entire first period under the control of the control section; and
a second recording processing section that, when a signal based on an operation by a user is provided in the first period, records a copy of the medical image in a second recording region in a predetermined second period before and after a timing of the operation by the user, under the control of the control section.

2. The medical information recording apparatus according to claim 1, wherein the second recording processing section merges with each other a plurality of copies of the medical image generated as a result of the operation being performed by the user a plurality of times.

3. The medical information recording apparatus according to claim 2,
wherein in the merging with each other of the plurality of copies, the second recording processing section inserts a transient image between the copies.

4. The medical information recording apparatus according to any one of claims 1 through 3,
wherein the first recording processing section records the medical image as one moving image file; and
wherein the second recording processing section records a copy of the medical image as one respective moving image file for each of the operations by the user.

5. A medical information recording apparatus comprising:
a control section that controls moving image recording of a plurality of medical images from a plurality of medical apparatuses;
a first recording processing section that records the plurality of medical images in regions for the respective medical images in a first recording region over an entire first period under the control of the control section; and
a second recording processing section that, when a signal based on an operation by a user is provided within the first period, records copies of the medical images in regions for respective medical images in a second recording region in a predetermined second period before and after a timing of the operation by the user, under the control of the control section.

6. The medical information recording apparatus according to claim 5, wherein the second recording processing section merges with each other a plurality of the second medical images generated as a result of the operation being performed by the user a plurality of times.

7. The medical information recording apparatus according to claim 6,
wherein in the merging with each other of the plurality of the second medical images, the second recording processing section inserts a transient image between the second medical images.

8. The medical information recording apparatus according to any one of claims 5 through 7,
wherein the first recording processing section records each of the first medical images as one moving image file; and
wherein the second recording processing section records each of the second medical images as one moving image file for each of the operations by the user.

9. The medical information recording apparatus according to any one of claims 1 through 8,
wherein the control section sets the first and second recording regions in respective different subfolders within a same folder.

10. The medical information recording apparatus according to any one of claims 1 through 9,
wherein the operation by the user is an operation of a release button provided at the medical apparatus.
